(19) Europäisches Patentamt
European Patent Office
Office européen des brevets

(11) Publication number: **0 142 732**
**B1**

(12) # EUROPEAN PATENT SPECIFICATION

(45) Date of publication of patent specification: **24.01.90**

(21) Application number: **84112758.2**

(22) Date of filing: **23.10.84**

(51) Int. Cl.[5]: **C 07 D 487/22,**
C 07 D 519/00, A 61 K 31/40
// (C07D487/22, 257:00, 209:00, 209:00, 209:00),(C07D519/00, 487:00, 487:00)

(54) **Pheophorbide derivatives and pharmaceutical preparations containing them.**

(30) Priority: **24.10.83 JP 198934/83**
**22.06.84 JP 128478/84**

(43) Date of publication of application:
**29.05.85 Bulletin 85/22**

(45) Publication of the grant of the patent:
**24.01.90 Bulletin 90/04**

(84) Designated Contracting States:
**CH DE FR GB IT LI**

(56) References cited:
**PATENTS ABSTRACTS OF JAPAN, vol. 7, no. 28 (C-149)1173r, 4th February 1983; & JP - A - 57 185 220 (YAKULT HONSHA K.K.) 15-11-1982**
**CHEMICAL ABSTRACTS, vol. 99, 12th September 1983, page 284, no. 84785q, Columbus, Ohio, US; J. BECK et al.: "Optically detected magnetic resonance of porphyrin complexes in the bacterium Rhodopseudomonas sphaeroides" & Z. NATURFORSCH., C: BIOSCI. 1983, 38C(3-4), 220-9**

**The file contains technical information submitted after the application was filed and not included in this specification**

(73) Proprietor: **TOYO HAKKA KOGYO KABUSHIKI KAISHA**
**75-1 Oaza Hamanaka**
**Satosho cho Asakuchi gun Okayama Pref. (JP)**

(72) Inventor: **Sakata, Isao**
**1766-4 Obirai**
**Kasaoka City Okayama Pref. (JP)**
Inventor: **Nakajima, Susumu**
**4-4-34 Gojo Midorigaoka**
**Asahikawa City Hokkaido (JP)**
Inventor: **Koshimizu, Koichi**
**856-10 Horensan soenishi cho**
**Nara City Nara Pref. (JP)**
Inventor: **Samejima, Natsuki**
**A-42 Idai Shukusha, 3-3 Nijo Midori-gaoka**
**Asahikawa City Hokkaido (JP)**
Inventor: **Inohara, Kazumi**
**3-9-7 Mikado cho**
**Fukuyama City Hiroshima Pref. (JP)**
Inventor: **Takata, Hiroyuki**
**2098 Satomi Satosho cho**
**Asakuchi gun Okayama Pref. (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 142 732 B1**

Courier Press, Leamington Spa, England.

(56) References cited:
CHEMICAL ABSTRACTS, vol. 82, 1975, page 203, no. 134838f, Columbus, Ohio, US; R.F. McFEETERS: "Substrate specificity of chlorophyllase" & PLANT PHYSIOL. 1975, 55(2), 377-81

TETRAHEDRON LETTERS, no. 44, 1979, pages 4257-4260, Pergamon Press Ltd., Oxford, GB; N. RISCH et al.: "Partialsynthese eines stereochemisch einheitlichen Bacteriochlorophylls D"

JUSTUS LIEBIGS ANNALEN DER CHEMIE, vol. 745, 1971, pages 87-98, Weinheim, DE; H. WOLF et al.: "Bestimmung der absoluten Konfiguration diastereomerer 9-Desoxo-9(R.S)-hydroxy-10(R.S)-methylphäophorbide der a-Reihe"

JOURNAL OF ORGANIC CHEMISTRY, vol. 43, no. 2, 1978, pages 281-283, American Chemical Society, Washington, US; P.A. ELLSWORTH et al.: "Methyl 10-epipheophorbide a: an unusual epimeric stability relative to chlorophyll a or a'1"

**Description**

The present invention relates to novel pheophorbide derivatives which are useful in cancer treatment. The compounds according to the present invention are novel and are extremely useful in cancer treatment because of their pharmacological effects such as photo-sensitivity and accumulative and destructive effects in cancer cells.

Porphyrin compounds, particularly porphine derivatives obtained from hemoglobin, are known for their binding property with cancer tissues and photo-dynamic characteristics. Their use in cancer treatment is now being studied. However, production of porphyrin compounds requires animal blood which is limited in supply and extensive production steps. Supply of the material animal blood, moreover, is not stable and porphine derivatives such as hematoporphyrin which are said to be the active constituent are unstable and it is extremely difficult to obtain them with high purity.

It has heretofore been known that porphine derivatives of hemoglobin origin, i.e. hematoporphyrin and diacetyl hematoporphyrin, are likely to be conjugated with cancerous tissues and are photo sensitive.

Endo et al have recently found that 10-hydroxy-pheophorbide is carcinostatic (Jap. Pat. Appln. Laid-open No. Sho 57—185220). This application discloses that pheophorbide and pyropheophorbide also have the same activities. Although these compounds are photo-sensitive (because they are porphyrin related compounds), they have no selectively accumulative effect in cancer cells. No data on their affinity to cancer cells are found in references nor is there any description concerning the differences in selective accumulation in cancer cells among the listed compounds other than the calculated results on the tumor suppression rate after administration of the substance and laser irradiation.

Yori et al reported on the bacterial effect of ethylenediamine hydrochloride of pheophorbide (Jap. Pat. Appln. Laid-open No. Sho 58—981). This application relates to an antibacterial agent comprising water soluble phorbine derived from pheophorbide by converting the same into ethylenediamine hydrochloride.

Processes for synthesizing chlorophyll dimers are disclosed in the literature (M. R. Wasielewski et al, C.A. *86,* 68532f(1977), C.A. *88* 148988r(1978) and S. G. Boxer et al, J.A.C.S., *1976,* 5406). Also disclosed in references is the synthesis of compounds such as 9-desoxo-9-hydroxy-pheophytin (M. R. Wasielewski et al., Tetrahedron Letters, 1043 (1978)) and 9-desoxo-9-hydroxy-10-methoxy-methylpheophorbide (H. Scheer et al., Tetrahedron, 28, 5839 (1972)). However, these substances are different from the compounds of the invention. Moreover, the aim of the authors of said articles was to synthesize the compounds as a part of their study in elucidating the mechanism of photo-synthesis in order to obtain compounds having an UV absorption profile more similar to that of chlorophyll P-700. The latter substance is the result of the study in which various related derivatives were synthesized to determine their absolute configuration, to investigate the influence of reaction conditions on yield and to publish data obtained from analyses and measurements by various devices. These references do not discuss physiological activities of the compounds on cancerous tissues which are the main object of the present invention.

The present inventors have long been engaged in the study of chlorophyll related compounds and established a highly economical process for separating pheophorbide using photo-synthesized organisms which are available in a stable and inexpensive supply. (Jap. Pat. Appln. Laid-Open No. Sho 58—69884).

It is the object of the present invention to provide novel pheophorbide-derivatives having more potent physiological activities than said porphines derived from blood. This object is achieved by the invention.

The subject matter of the invention are novel pheophorbide derivatives having various functional groups expressed by general formula (I) and their dimers expressed by general formula (II) (hereinafter referred to as the present invention substances), excluding pheohorbide, pyropheophorbide, and 2-desethenyl-2-(1-hydroxyethyl)-pheophorbide.

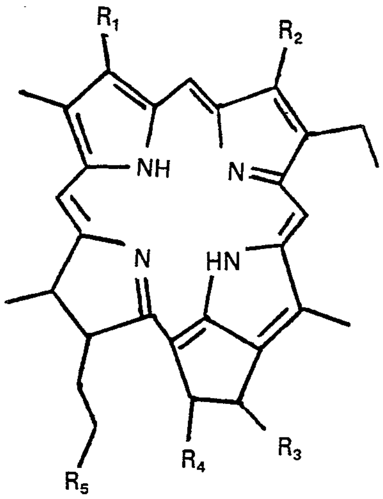

(I)

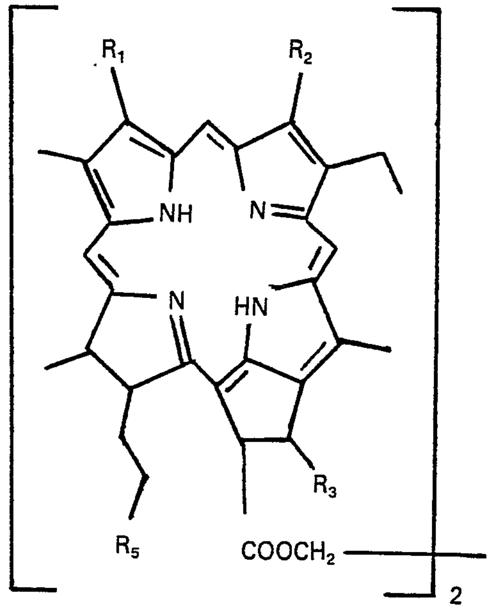

(II)

wherein $R_1$ represents $CH{=}CH_2$,

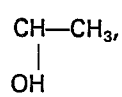

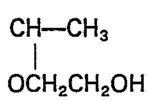

or

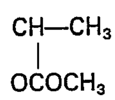

$R_2$ represents $CH_3$, CHO or $CH_2OH$,
$R_3$ represents $=O$, OH or $OCOCH_3$
$R_4$ represents H,$COOCH_3$, $COOCH_2CH_2OH$, $COOCH_2CH_2OCOCH_3$ or a CONH— residue of an amino acid and
$R_5$ represents COOH, COO alkali, and, if $R_4$ represents a CONH-residue of an amino acid, $R_5$ may in addition represent $COOCH_3$.

Preferred compounds of the invention are 9-desoxo-9-hydroxy-pheoboride derivatives and alkaline salts thereof represented by the general formula (Ia):

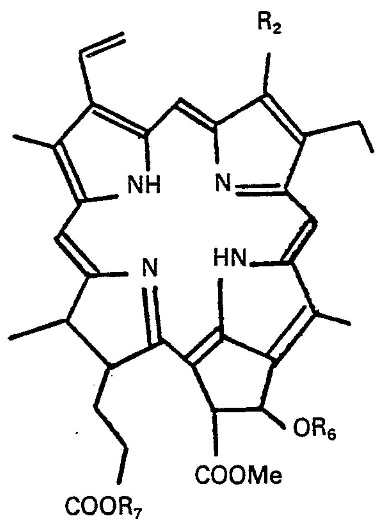

(Ia)

wherein
$R_2$ represents $CH_3$ or $CH_2OH$,
$R_6$ represents H or Ac, and
$R_7$ represents H or an alkali metal such as Na and K.

Results of elemental analysis and nuclear magnetic resonance absorption of 9-desoxo-9-hydroxy-pheophorbide which is one of the preferred compounds of the invention are as follows:

Formula: $C_{35}H_{38}N_4O_5$
Elementary analysis:
Calcd. C, 70.69%: H, 6.44%: N, 9.42%
Found: C, 70.64%: H, 6.57%: N, 9.16%

Nuclear magnetic resonance absorption ($^1H$—NMR) (δ ppm)

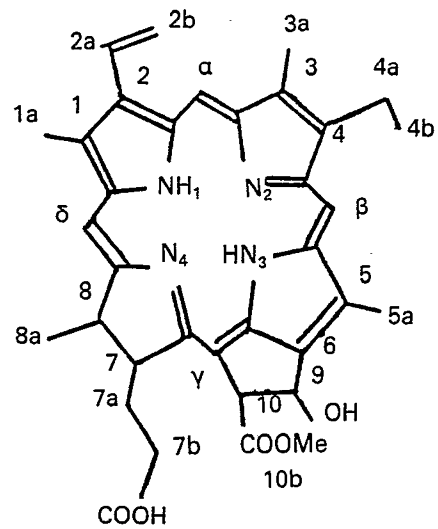

| | |
|---|---|
| α — H : 9.49 | 7 — H : 4.1 |
| β — H : 9.68 | 7a — $CH_2$ : 2 ~ 3 |
| δ — H : 8.82 | 7b — $CH_2$ : 2 ~ 3 |
| 1 — Me : 3.44 | 8 — H : 4.35 |
| 2 — Vinyl : 8.00 | 8 — Me : 1.75 |
| : 5.89 | 9 — H : 6.48 |
| : 6.03 | 10 — H : 6.26 |
| 3 — Me 3.23 | 10b — COOMe : 3.70 |
| 4b — Me : 1.59 | N — H : 0.33 |
| 5 — Me : 3.61 | : −0.28 |

The compounds of the invention have excellent pharmacological properties such as marked affinity to cancerous tissues, high photo-sensitivity and destructive effect on cancerous tissues that are superior to those observed in porphines. It has so far been unknown that the phorbine related compounds had such strong physiological activities. The inventors often observed this fact during the course of their study, which is surprising as, for example, no specific activities against cancer were found in the raw material of the invention substances, that is, pheophorbide and its alkaline salts and methyl pheophorbide.

The invention will now be further explained with respect to the drawing in which

Fig. 1 and 2 are graphs showing the affinity of compounds according to the invention to cancer cells as measured by $N_2$ pulsed laser spectrofluorometry;

Fig. 3 and 4 are graphs showing the destructive effect of said compounds on cancer cells as measured by biochemiluminescence in a single photon-counter system.

Pharmacological activities of the present invention substances will now be explained. The present invention substances accumulate selectively in cancer tissues and only slowly excrete therefrom. When irradiated with light, they reaact and produce singlet oxygen having a strong oxidizing effect to thereby destroy cancer cells. As the present invention substances are readily excreted from normal organs and cells, cancer can thus be treated without damage to normal cells.

It is possible to treat not only the cancer projecting on the surface but also small cancer lesion in the

mucous epithelium which is difficult to find and abnormal cells in precancer state.

As for effects on cancer, most of porphines, except for a very few substances, show no selective affinity to cancer cells although they are photo-sensitive. Absence of selectivity leads to destruction of normal cells as well as cancer cells.

In view of the above facts, there has been proposed a combined use of several porphines having selective affinity.

The present invention substances are of great utility as they are provided with all the properties mentioned above (i.e., affinity to cancer cells, photo-sensitivity, destructive effect on cancer). These substances can be used not only singly but in combination.

The process for preparing the compounds according to the present invention will now be described. Roughly, there are two ways of preparing the invention compounds having the general formula I. According to one process pheophorbide (1) is treated with hydrobromic acid to obtain 2-desethenyl-2-(1-hydroxyethyl)-pheophorbide (2), which is then reduced to obtain 2-desethenyl-2-(1-hydroxyethyl)-9-desoxo-9-hydroxy-pheophorbide (3). (3) is acetylated to obtaion diacetylate (4). The reactions can be expressed by the following chemical formula:

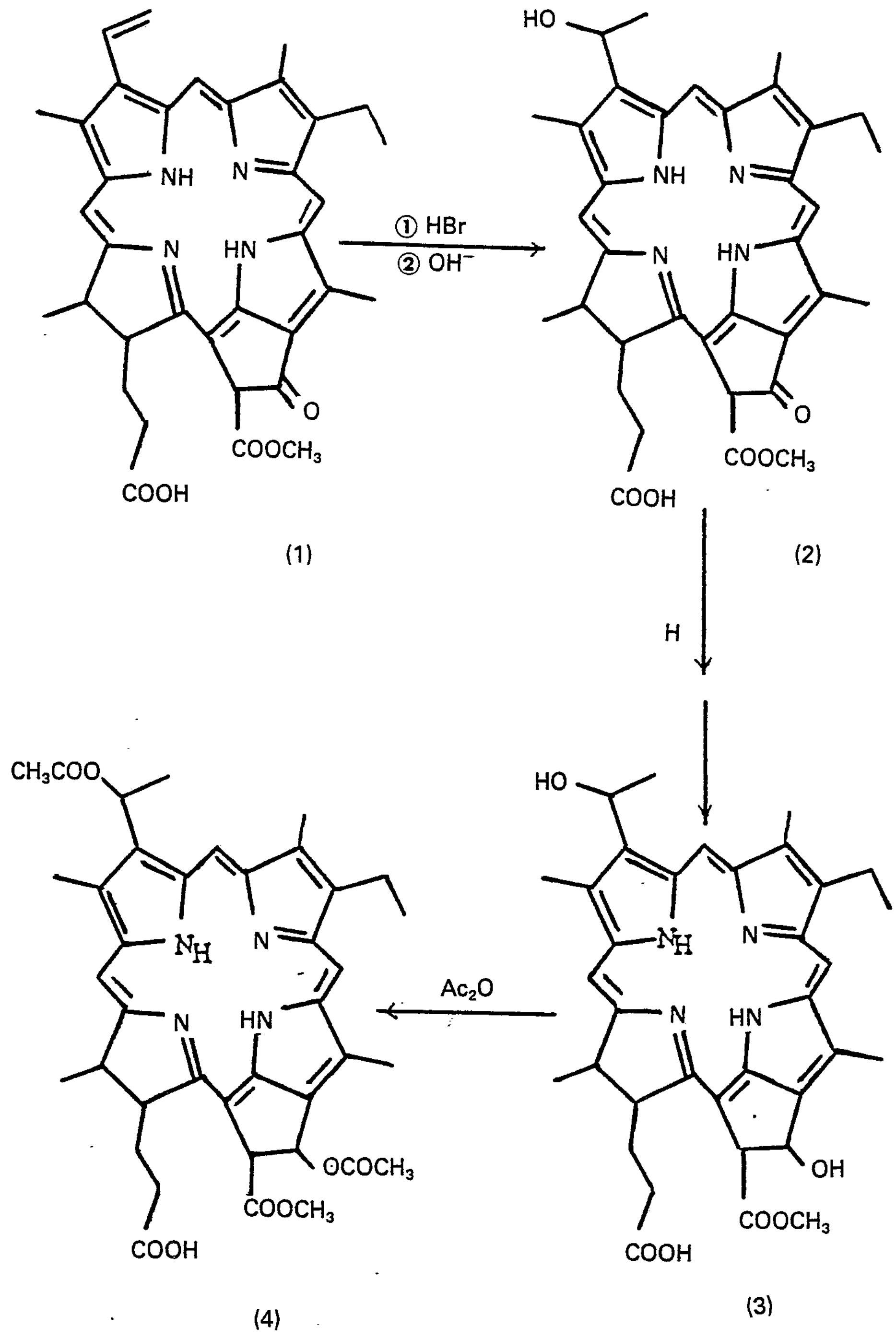

In practical application of the present invention, (1) is dissolved in hydrobromic acid/acetic acid, reacted and neutralized to obtain (2). (2) is reacted using a suitable reducing agent such as $NaBH_4$ to obtain the product (3). Diacetylated product (4) can be obtained from (3) by reaction with acetic anhydride/pyridine in the usual way.

According to another process for preparing the compounds of the general formula I, methylpheophorbide (5) is esterexchanged with ethylene glycol to obtain ethylene glycol mono-10b-methyl pheophorbate (6), or (5) is ester-exchanged with phenylalanine t-butyl ester to obtain N-phenylalanine t-butyl ester 10b-methylpheophorbidamide (7). (6) and (7) are subjected to acid hydrolysis to obtain ethylene glycol mono-10b-pheophorbate (8) and N-phenylalanine 10b-methyl pheophorbidamide (9). The reactions can be expressed by the following chemical formula:

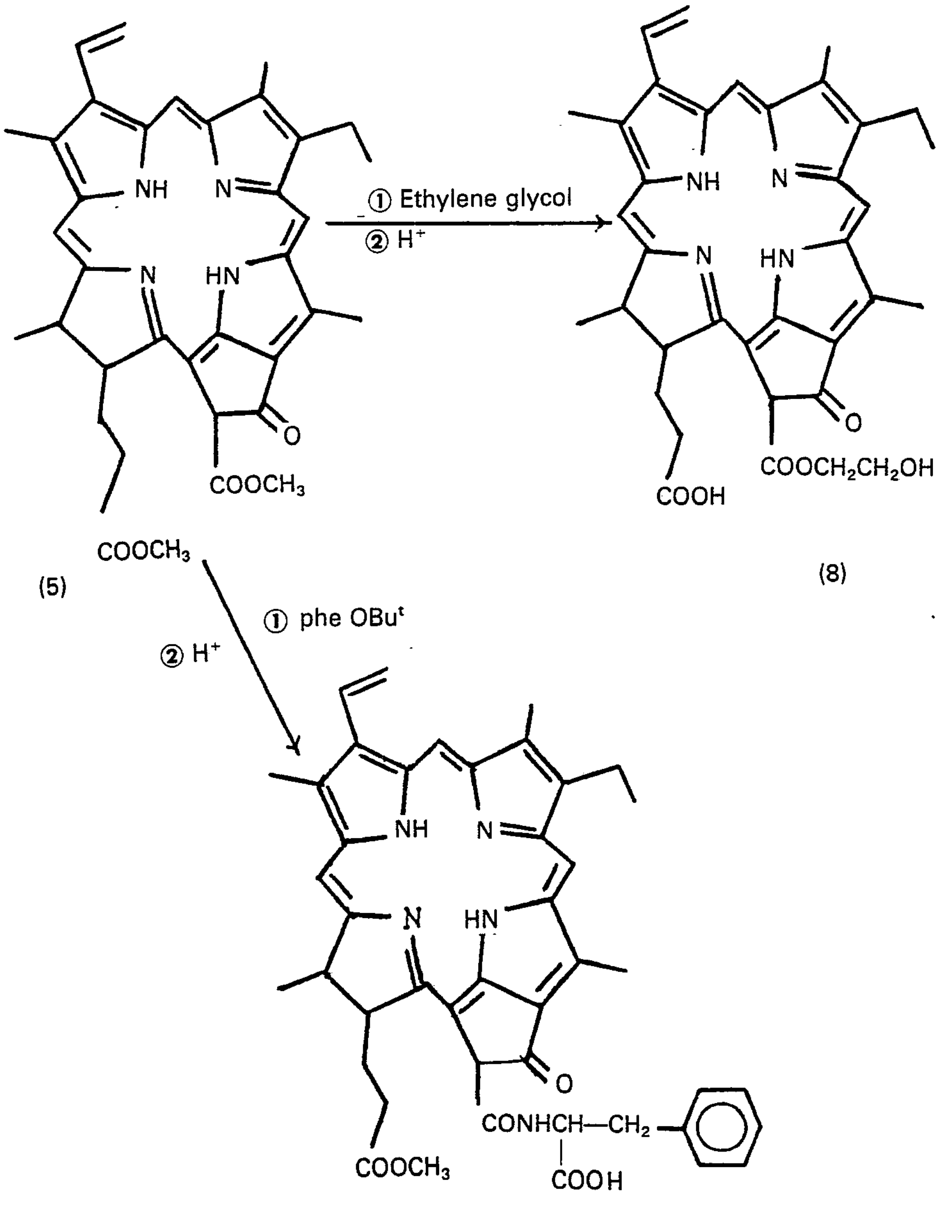


(5)

(8)

(9)

Hydroxides, reduced derivatives or acetylates of (8) and (9) may be produced by subjecting said esters of pheophorbide to hydrobromic acid treatment, reduction or acetylation as mentioned above.

In practical application of the present invention, (5) is dissolved in a suitable solvent such as xylene or DMF, ethylene glycol is added and the mixture is heated and reacted to obtain (6). Alternatively, phenylalanine t-butyl ester is added in place of ethylene glycol, heated and reacted to obtain (7). The resultant product (6) is reacted with 50% $H_2SO_4$ and the product (7) with $CF_3COOH$ respectively to obtain (8) and (9).

The dimers of the present invention having the general formula II are obtained by condensating (8) with (1) through ester-exchange to obtain ethylene glycol dio-10b-pheophorbate (10). The reaction can be expressed by the following formula:

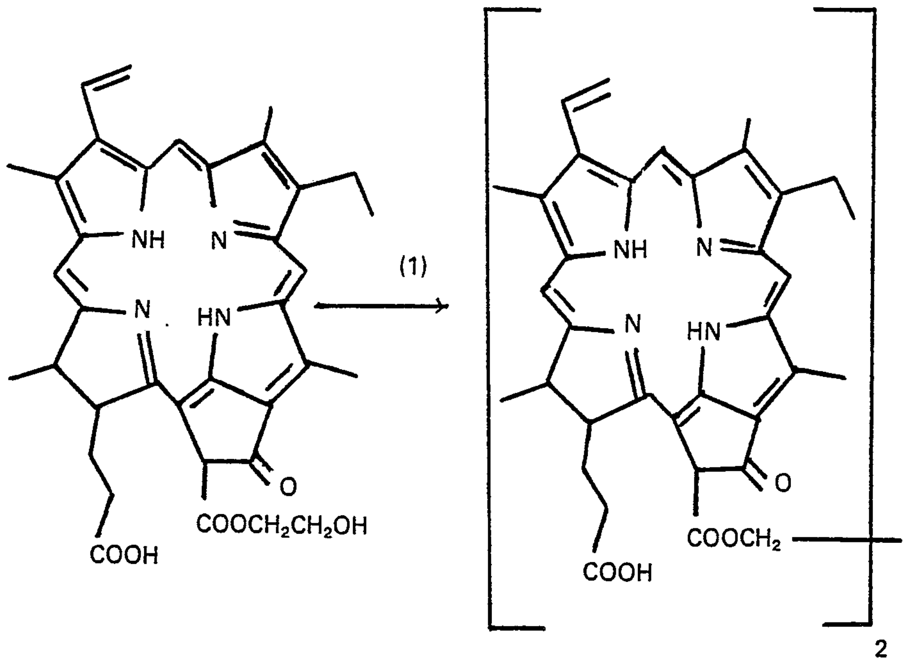


(8) (10)

Said pheophorbide dimer (10) can be subjected to hydrobromic acid treatment, reduction or acetylation in the same manner as mentioned above to produce hydroxides, reduced derivatives or acetylates of 10, respectively.

In practical application of the present invention, (8) may be dissolved in a suitable solvent such as DMF or DMA. Then (1) is added and the mixture is heated and reacted to obtain (10).

As a still another process or preparing the present invention substances, it is possible to form the hydroxide or reduced derivatives of methyl pheophorbide (5) prior to condensation by ester-exchange, and obtain preferable results. Any combination of reactions other than those mentioned above may also be employed so long as they include hydroxidization, hydrolysis and ester-exchange reactions. It is also preferable but not essential to add a suitable antioxidant or solvent and to heat the reaction system. Although pheophorbide is used as the starting material, pheophorbide, a, b, c, pyropheophorbide and pheophytin may also be used. The invention is, however, not limited to those so long as the material is obtained from a photo-synthesized substance.

The preferred compounds of general formula Ia can be obtained by reducing pheophorbide (1) to produce 9-desoxo-9-hydroxy-pheophorbide (11). The reaction can be expressed by the following chemical formula:

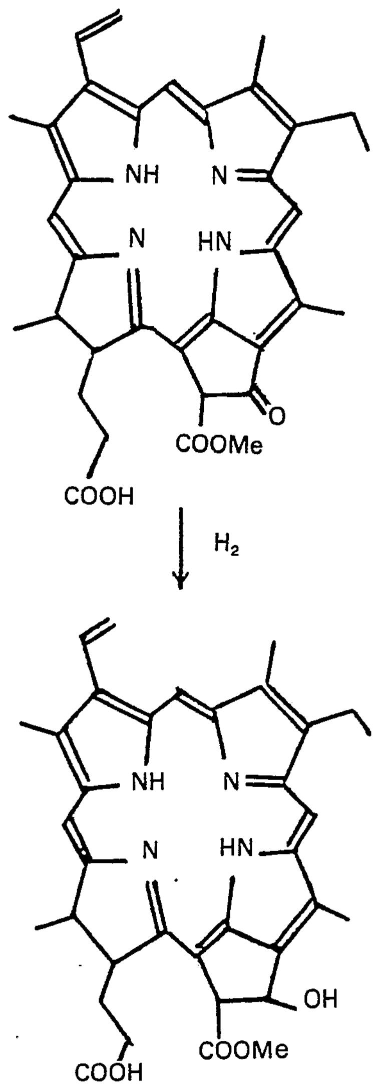


(1)

(11)

In practical application of the present invention, (1) is dissolved in a suitable solvent, i.e. methanol, and reacted with a suitable reducing agent such as $NaBH_3$.

A preferable result can be obtained by another preparation method of the present invention substances, wherein pheophytin or methyl pheophorbide is reduced in advance in the first step and the reaction product, 9-desoxo-9-hydroxy-pheophytin or 9-desoxo-9-hydroxy-methyl pheophorbide is hydrolyzed in the second step.

A similarly preferable result can be obtained by still another process; pheophorbide obtained through acid hydrolysis of pheophytin is similarly reduced to obtain (11), or (11) is acetylated to produce 9-desoxo-9-acetyloxy-pheophorbide (12) because when (12) is an alkaline salt, it is released prior to (11). Any combination of reactions other than those mentioned above may also be employed so long as they include hydrolysis, reduction and acetylation. It is also preferable but not essential to add a suitable antioxidant or solvent and to heat the reaction system.

Although methanol, ethanol, acetonitrile, pyridine, etc. as solvents and $Na_2S_2O_3$, $Na_2S_2O_4$, ascorbic acid, etc. as reducing agents may be employed in preparing the present invention substances, the invention is not restricted to these solvents and reducing agents.

Table I illustrates data on Nuclear Magnetic Resonance absorption spectrum of the present invention substances.

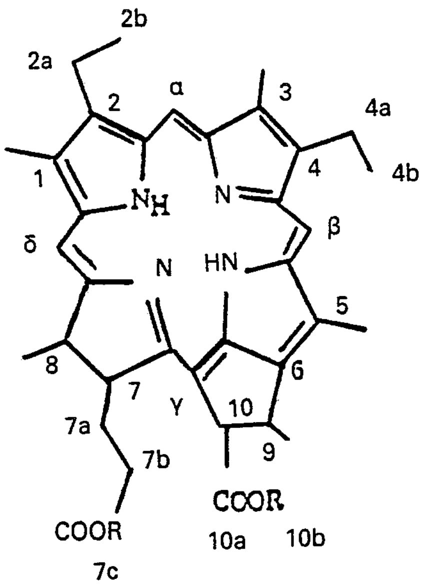

TABLE I (δ ppm)

| | 1 | 2 | 3 | 5 | 6 | 7 | 8 | 9 | 10 |
|---|---|---|---|---|---|---|---|---|---|
| Mw | 592 | 610 | 612 | 606 | 636 | 795 | 622 | 739 | 1182 |
| α-H | 9.05 | 8.85 | 8.70 | 9.16 | 9.00 | 9.07 | 9.02 | 9.08 | 9.12 |
| β-H | 9.22 | 9.25 | 9.00 | 9.36 | 9.31 | 9.30 | 9.29 | 9.32 | 9.19 |
| δ-H | 8.43 | 8.29 | 9.32 | 8.53 | 8.42 | 8.50 | 8.43 | 8.48 | 8.40 |
| 1-$CH_3$ | 3.26 | 3.25 | 3.15 | 3.30 | 3.22 | 3.25 | 3.38 | 3.26 | 3.37 |
| 2-$CH=CH_2$ | 7.72 | — | — | 7.83 | 7.70 | 7.73 | 7.71 | 7.72 | 7.80 |
| | 6.05 | — | — | 6.15 | 6.10 | 6.10 | 6.10 | 6.10 | 6.15 |
| | 5.95 | — | — | 6.06 | 5.93 | 5.98 | 6.01 | 5.95 | 5.99 |
| 3-$CH_3$ | 3.00 | 3.06 | 3.00 | 3.04 | 2.95 | 2.99 | 3.17 | 2.98 | 3.14 |
| 4b-$CH_3$ | 1.62 | 1.63 | 1.68 | 1.60 | 1.53 | 1.58 | 1.66 | 1.58 | 1.68 |
| 5-$CH_3$ | 3.57 | 3.43 | 3.22 | 3.60 | 3.52 | 3.52 | 3.62 | 3.52 | 3.59 |
| 7-H | 4.13 | 4.2 | 4.1 | 4.16 | 4.2 | 4.4 | 4.2 | 4.3 | 4.2 |
| 7a,b-$CH_2CH_2$— | 2~3 | 2~3 | 2~3 | 2~3 | 2~3 | 2~3 | 2~3 | 2~3 | 2~3 |
| 7c-$CH_3$ | — | — | — | 3.56 | 3.60 | 3.61 | — | 3.60 | — |
| 8-H | 4.40 | 4.7 | 4.4 | 4.44 | 4.5 | 4.55 | 4.4 | 4.53 | 4.43 |
| 8-$CH_3$ | 1.86 | 1.90 | 1.71 | 1.81 | 1.80 | 1.84 | 1.85 | 1.82 | 1.85 |
| 9-H | — | — | 6.69 | — | — | — | — | — | — |
| 10-H | 6.17 | 6.11 | 6.13 | 6.25 | 6.21 | 6.13 | 6.22 | 6.15 | 6.24 |
| 10b-$CH_3$ | 3.85 | 3.88 | 3.42 | 3.88 | — | — | — | — | — |
| 10b-$CH_2CH_2$— | — | — | — | — | 3.93 | — | 4.15 | — | — |
| 10b-$CH_2$— | — | — | — | — | — | — | — | — | 4.2 |
| 10b-$OBu^t$ | — | — | — | — | — | 1.40 | — | — | — |
| 10b 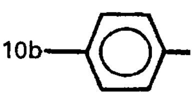 $CH_2$— | — | — | — | — | — | 7.2 | — | 7.2 | — |

As mentioned above, the present invention utilizes photosynthesized substances as the raw material which are inexpensive and stably supplied. The process involves extremely simple operaations which can be conducted in a short period of time to achieve isolation and synthesis. The present invention substances have physiological activities which are unique to phorbine related compounds and can be used per se as medical preparations, or can be formulated to medical preparations in a usual manner. Unlike porphines derived from hemoglobin, they can be mass produced at higher purity and thus are extremely useful.

The preparation of the compounds of the present invention will now be described by way of examples.

Example 1

Three grams of phoephorbide (1) were disoslved in 12 ml of pyridine and 60 ml of methanol into which 5% $NaBH_4$ solution was dropped under stirring. After the reaction was completed, crystals of 9-desoxo-9-hydroxy-pheophorbide (11) were precipitated after addition of an aqueous solution of 5% citric acid. Crystals were collected, washed with water and dried. (Yield 3 g). The yield was 98%.

The product obtained was dissolved in acetone and neutralized by adding 5% $Na_2CO_3$ aqueous solution to obtain sodium 9-desoxo-9-hydroxy pheophorbide. The yield was 98%.

Example 2

Three grams of methyl pheophorbide (5) was dissolved in 70 ml of acetonitrile, an aqueous solution of $NaBH_4$ was added dropwise under stirring to obtain crude 9-desoxo-9-hydroxy-methyl pheophorbide.

The product obtained was suspended and dissolved in 40% $H_2SO_2$ aqueous solution and reacted for 1 hour under agitation. After the reaction was completed, 5% citric acid aqueous solution was added to the reaction liquid by a similar process as in Example 1 to collect the product (11), which was washed with water and dried. The yield was 69%.

Example 3

Fifty grams of crude chlorophyll (chlorophyll content 10%) was suspended and dissolved in 3-fold 50% $H_2SO_4$ aqueous solution, 0.5 g of $Na_2S_2O_3$ was added, and the mixture was reacted for 1 hour under agitation. Similar process as in Example 2 was conducted to obtain (1), which was further treated as in Example 1 to obtain (11) (yield 2.5 g). The yield (in proportion to crude chlorophyll used) was 7.6%.

Example 4

One gram of (11) was acetylated with acetic anhydride-pyridine by a conventinal method to obtain 9-desoxo-9-acetyloxy-pheophorbide (yield 1 g). The yield was 99%:

0.5 g of this product was dissolved in ether and neutralized with 5% $Na_2CO_3$ aqueous solution to obtain the sodium salt of 9-desoxo-acetyloxy-pheophorbide. The aqueous solution of this sodium salt was left standing for reaction to obtain 0.2 g of (11). The yield was 40%.

Example 5

One gram of pheophorbide (1) was dissolved (1) was dissolved in 25 g of hydrobromic acid/acetic acid and reacted for 15 hours under stirring. After reaction was completed, 50 ml of water was added under stirring and then neutralized by adding 20 ml of 33% NaOH aqueous solution, whereby crystals of 2-desethenyl-2-(1-hydroxyethyl)-pheophorbide (2) were precipitated. Crystals were then collected, washed with water and dried (yield: 1 g). The yield was 97%.

The resultant product (2) was dissolved in aceton, added with 5% $Na_2CO_3$ aqueous solution for neutralization to obtain sodium 2-desethenyl-2-(1-hydroxyethyl)pheophorbide. The yield was 98%.

Example 6

One gram of (2) was dissolved in 6 ml of pyridine and 30 ml of methanol, 5% $NaBH_4$ aqueous solution was dropped into the mixture under stirring and reacted. After the reaction was completed, 5% aqueous solution of citric acid was added to separate excess $NaBH_4$. Water was then added to the reaction product and crystals of 2-desethenyl-2-(1-hydroxyethyl)-9-desoxo-9-hydroxy-pheophorbide (3) were precipitated. Crystals were collected, washed with water and dried. (yield 1 g). The yield was 98%.

Example 7

One gram of (3) was acetylated using acetic anhydride/pyridine by the conventional method to obtain diacetylate (4) of (3) (yield 1.1 g). The yield was 99%.

Example 8

One gram of methyl pheophorbide (5) and 2 g of ethylene glycol were dissolved in 20 ml of DMF and reacted for 2 hours under heating and stirring. After the reaction was completed, water was added to precipitate crystals of ethylene glycol mono-10b-methyl pheophorbate (6). Crystals were collected, washed with water and dried (yield 0.7 g). The yield was 70%.

The product (0.7 g) thus obtained was dissolved in 10 ml of 50% $H_2SO_4$ and reacted for 1 hour at room temperature under stirring. After the reaction was completed, water was added to precipitate crystals of ethylene glycol mono-10b-pheophorbate (8). Crystals were collected, washed with water and dried (yield 0.65 g). The yield was 95%.

Example 9

One gram of (5) and 2 g of phenylalanine t-butyl ester were dissolved in 30 ml of xylene and reacted for 2 hours under heating and stirring. After the reaction was completed, the reaction solution was subjected to column chromatography (silicic acid column, n-hexane: ethyl acetate = 3:1) to obtain N-phenylalanine-t-butyl ester 10b-methylpheophorbid amide (7) (yield 0.9 g). The yield was 68%.

The resultant product (0.9 g) was dissolved in 10 ml of trifluoroacetic acid and reacted for 2 hours at room temperature under stirring. After the reaction was completed, water was added to precipitate crystals of N-phenylalanine 10b-methylpheophorbidamide (9). Crystals were collected, washed with water and dried (yield 0.8 g). The yield was 95%.

Example 10

One gram of (8) and 1 g of (1) were dissolved in 20 ml of DMF and reacted for 2 hours under heating and stirring. After the reaction was complėted, water was added to precipitate crystals of ethylene glycol di-10b-pheophorbate (10). Crystals were collected, washed with water and dried (yield 1.5 g). The yield was 80%.

The pharmacological effect of the compounds of the invention is shown by the following tests of laser irradiation on a removed organ in vitro.

A) $N_2$ Pulsed Laser Spectro-Fluorometry

Experiment 1

Five mg of the test drug sodium 9-desoxo-9-hydroxy pheophorbide diluted in physiological saline (1 ml) was administered (1 ml) to golden hamsters (5 each for a group) 14—21 days after transplantation of nitrosamine-induced cancer cells of the pancreas, after which cancer cells and other organs were removed. $N_2$-pulsed laser ($N_2$ 337 nm, 2 ns 400—1000 nm) was irradiated to each organ thus obtained to measure $N_2$ pulsed laser spectro-fluorometry. The result is shown in Fig. 1.

$N_2$ pulsed laser spectro-fluorometry of each removed organ was measured 24 hours and 48 hours afterwards to investigate the wavelengths of 600 to 900 nm based on the peak wavelengths of NADH of 470 nm.

Fig. 1 shows that though sodium 9-desoxo-9-hydroxy pheophorbide was generally found in organs after 24 hours, it had markedly accumulated in cancer cells alone but not in other cells after 48 hours.

As is obvious from the above result, sodium 9-desoxo-9-hydroxy pheophorbide was proved to have remarkable selective affinity to cancer cells.

Experiment 2

Golden hamsters (5 per one group) 14—21 days after transplantation of nitrosamine-induced cancer cells of the pancreas were administered 5 mg each of sodium 2-desethenyl-2-(1-hydroxyethyl) pheophorbide (sodium salt of (2)), sodium 2-desethynyl-2-(1-hydroxyethyl)-9-desoxo-9-hydroxy pheophorbide (sodium salt (3)), sodium 2-desethenyl-2-(1-acetyloxyethyl)-9-desoxo-9-acetyloxy pheophorbide (sodium salt of (4)), sodium ethylene glycol mono-10b-pheophorbate (sodium salt of (8)), disodium ethylene glycol di-10b-pheophorbate (sodium salt of (10)) and sodium N-phenylalanine 10b-methyl pheophorbid amide (sodium salt of (9)) respectively diluted with physiological saline (1 ml). Cancer cells and other organs were removed, irradiated with $N_2$-pulsed laser ($N_2$, 337 nm, 2 ns 400—1000 nm) to measure $N_2$ pulsed laser spectro fluorometry. Using the NADH peak wavelength of 470 nm as the reference, wavelengths in the range of from 600—900 nm were studied.

Fig. 2 shows that these products showed marked accumulation in cancer cells 24 hours after administration, but left no trace in other cells.

Similar tests were conducted on other products and the results are shown in Table II. Various organs were removed 24 hours after administration and their $N_2$ pulsed laser spectro-fluorometry was measured. Using the NADH peak wavelength of 470 nm as the reference, the wavelengths were calculated and shown in Table II.

As is evident from the result above, these phorbine related compounds have a marked selective affinity to cancer cells.

B) Biochemical luminescence by single photoncounter

Experiment 3

Sodium 9-desoxo-9-hydroxy pheophorbide dissolved in physiological saline of respective concentrations (25 µg/2 ml, 50 µg/2 ml, 100 µg/2 ml) was added to leukemic Molt 4 Cell ($1 \times 10^7$ Cells) and the resultant product was incubated for 24 hours at 37°C.

Each incubated cell thus obtained was irradiated with laser (He-Ne gas, 630 nm, 10 min, 20 mW) and trace biochemiluminescence was measured by single photoncounter system.

Intensity of biochemiluminescence substantially correponds to the degree of destructive effect on cancer cells and thus by studying the biochemiluminescence by single photoncounter system, it is possible to predict the destructive effect on cancer cells.

Fig. 3 shows the intensity of biochemiluminescence and the destructive effect on cancer cells after laser irradiation. It is seen from Fig. 3 that more than 95% of the cancer cells were destroyed when the subject substance in the amount of 100 µg/2 ml was used.

The foregoing result shows that sodium 9-desoxo-9-hydroxy pheophorbide has intense photo-sensitivity and remarkable destructive effect on cancer cells.

Experiment 4

Leukemic Molt 4 cell ($1 \times 10^7$ cells) was added with physiological saline containing sodium 2-desethenyl-2-(1-hydroxyethyl) pheophorbide (sodium salt of (2)) in respective concentrations (25 µg/2 ml, 50 µg/2 ml, 100 µg/2 ml) and then incubated (37°C, 24 hours). Incubated cells each were irradiated with laser

(He-He gas, 630 nm, 10 min, 20 mW) to investigate generation of biochemiluminescence by single photoncounter system by measuring biochemiluminescence. The intensity of biochemiluminescence by single photoncounter system substantially corresponds to the degree of destruction of the cancer cells. Therefore, by observing generation of biochemiluminescence by single photoncounter system, destruction of cancer cells may be predicted.

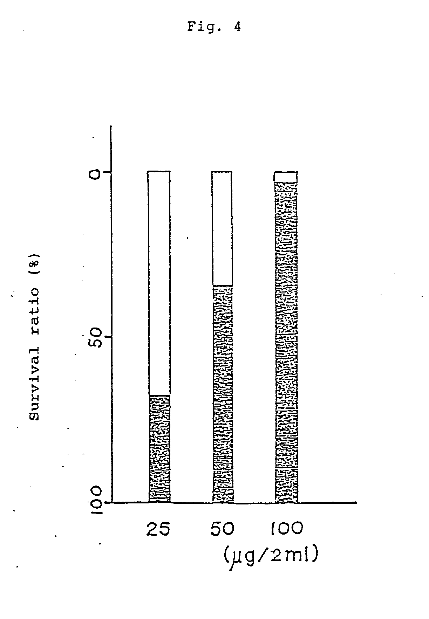

Fig. 4 shows the degree of biochemiluminescence and the destruction of cancer cells after laser irradiation. The result indicates that when 100 µg/2 ml of a test substance was used, more than 95% of the cancer cells were destroyed.

As is evident from the results shown in Fig. 4, phorbine related compounds are highly photo-sensitive and display a marked destructive effect on cancer cells.

The foregoing experiments A and B together prove that the present invention substances have a marked affinity to cancer cells, remarkable photo-sensitivity, and destructive effect on cancer cells.

TABLE II (Sodium compounds)

| | |
|---|---|
| **Comparison** | |
| Pheophorbide (1) | 0.78 |
| Pyropheophorbide | 0.25 |
| **Application** | |
| 9-Desoxo-9-hydroxy-pheophorbide+ethylene glycol mono-10b-methylpheophorbate (6) | 2.00 |
| 9-Desoxo-9-hydroxy-pyropheophorbide | 0.98 |
| 9-Desoxo-9-hydroxy-pheophorbide C | 0.32 |
| 9-Desoxo-9-acetyloxy-pheophorbide | 0.89 |
| N-Phenylalanine 10b-methylpheophorbidamide (9) | 0.75 |
| 2-Desethenyl-2-(1-acetyloxyethyl)-pheophorbide | |
| 2-Desethenyl-2-(1-hydroxyethyl)-9-desoxo-9-hydroxy-pheophorbide (3) | 3.13 |
| 2-Desethenyl-2-(1-acetyloxyethyl)-9-desoxo-9-acetyloxypheophorbide (4) | 0.48 |
| 2-Desethenyl-2-(1-acetyloxyethyl)-9-desoxo-9-hydroxy-pheophorbide | 0.95 |
| N-Phenylalanine 10b-9-desoxo-9-hydroxy-methylpheophorbidamide | 0.73 |
| Ethylene glycol di-10b-pheophorbate (10) | 2.78 |
| Ethylene glycol di-10b-9-desoxo-9-hydroxypheophorbate | 2.50 |
| Ethylene glycol mono-10b-pheophorbate (8) | 1.72 |
| Monoacetylethylene glycol mono-10b-pheophorbate | 1.30 |
| Ethylene glycol mono-10b-9-desoxo-9-hydroxy-pheophorbate | 2.10 |
| Monoacetylethylene glycol mono-10b-9-desoxo-9-acetyloxy-pheophorbate | 0.85 |
| Monoacetylethylene glycol mono-10b-9-desoxo-9-hydroxy-pheophorbate | 1.20 |
| 2-Desethenyl-2-(1-hydroxyethyl)-pheophorbide (2) | 1.90 |
| Ethylene glycol di-10b-2-desethenyl-2-(1-hydroxyethyl)-pheophorbate | 2.50 |
| Ethylene glycol di-10b-2-desethenyl-2-(1-hydroxyethyl)-9-desoxo-9-hydroxy-pheophorbate | 1.80 |
| 9-Desoxo-9-hydroxy-pheophorbate (11) | 0.93 |

**Claims**

1. Pheophorbide derivatives and alkali metal salts thereof represented by the general formula I and their dimers and alkali metal salts thereof represented by the general formula II

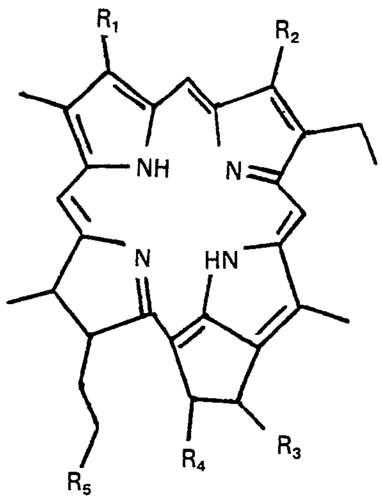

(I)

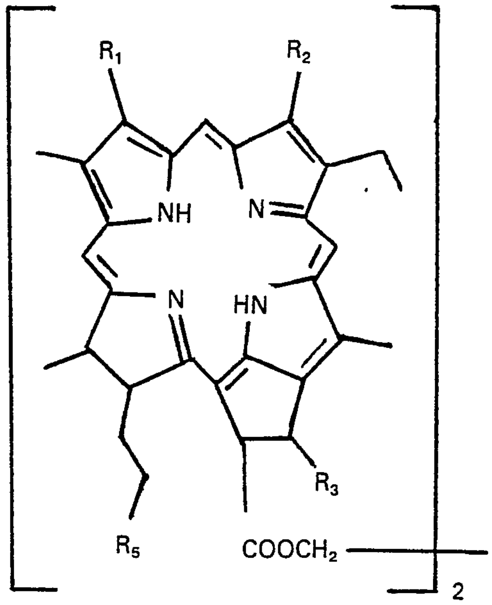

(II)

wherein $R_1$ represents $CH=CH_2$,

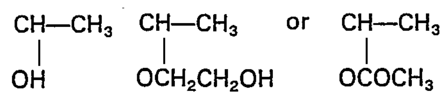

or

$R_2$ represents $CH_3$, CHO or $CH_2OH$,
$R_3$ represents =O, OH or $OCOCH_3$,
$R_4$ represents H, $COOCH_3$, $COOCH_2—CH_2OH$ $COOCH_2CH_2OCOCH_3$ or a CONH— residue of an amino acid, and
$R_5$ represents COOH, COO alkali and if $R_4$ represents a CONH residue of an amino acid, $R_5$ may in addition represent $COOCH_3$ provided, however, said derivatives and alkaline salts exclude pheophorbide, pyropheophorbide and 2-desethenyl-2-(1-hydroxyethyl)-pheophorbide.

2. Pheophorbide derivatives and alkali metal salts thereof according to claim 1 represented by the general formula Ia

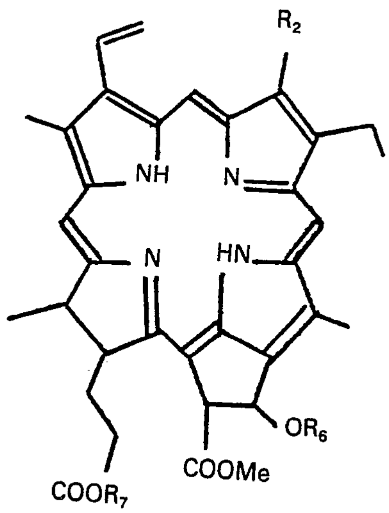

(Ia)

wherein
$R_2$ represents $CH_3$ or $CH_2OH$,

$R_6$ represents H or Ac, and

$R_7$ represents H or an alkali metal such as Na and K.

3. Pheophorbide derivatives having the general formula I, Ia or II as claimed in claims 1 or 2 including 2-desethenyl-2-(1-hydroxyethyl)-pheophorbide for use as a medicament.

4. Pheophorbide derivatives having the general formula I, Ia or II as claimed in claims 1 or 2 including 2-desethenyl-2(1-hydroxyethyl)-pheophorbide for use in the treatment of cancer.

5. Pharmaceutical compositions containing a pheophorbide derivative having the general formula I, Ia or II as claimed in claims 1 or 2 including 2-desethenyl-2-hydroxyethyl)-pheophorbide and a pharmaceutically acceptable carrier.

**Patentansprüche**

1. Pheophorbid-Derivate und Alkalimetallsalze davon der allgemeinen Formel I und ihre Dimeren und Alkalimetallsalze davon der allgemeinen Formel II

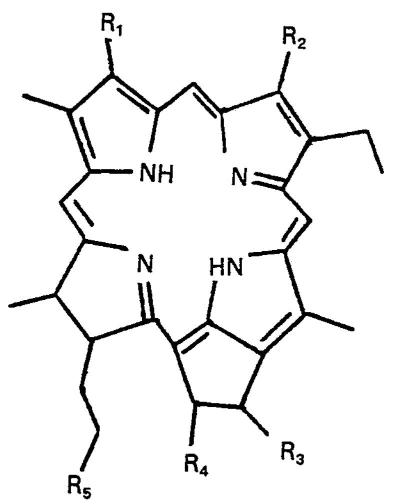

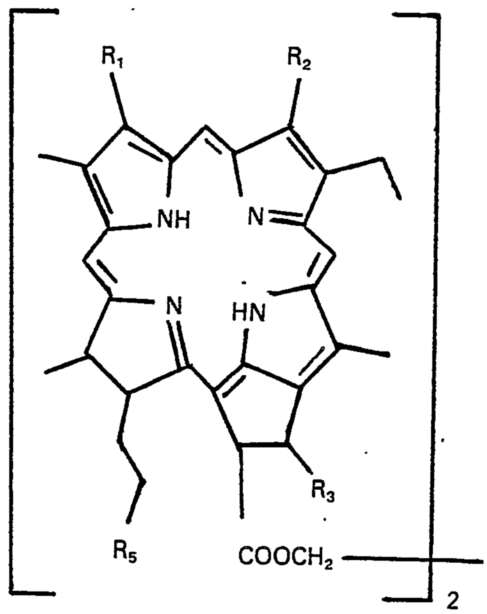

(I) (II)

in denen $R_1$ eine der Gruppen

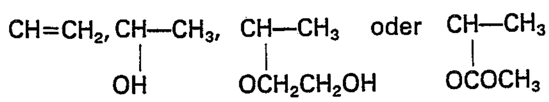

darstellt,

$R_2$ $CH_3$, CHO oder $CH_2OH$ bedeutet,

$R_3$ =O, OH oder $OCOCH_3$ bedeutet,

$R_4$ H, $COOCH_3$, $COOCH_2$—$CH_2OH$, $COOCH_2CH_2OCOCH_3$ oder einen CONH-Rest einer Aminosäure bedeutet, und $R_5$ COOH, COO-Alkali darstellt, und wenn $R_4$ einen CONH-Rest einer Aminosäure bedeutet, $R_5$ zusätzlich $COOCH_3$ bedeuten kann, jedoch mit der Maßgabe, daß die Derivate und Alkalisalze Pheophorbid, Pyropheophorbid und 2-Desethenyl-2-(1-hydroxyethyl)pheophorbid nicht einschließen.

2. Pheophorbid-Derivate und Alkalimetallsalze davon nach Anspruch 1 der allgemeinen Formel Ia

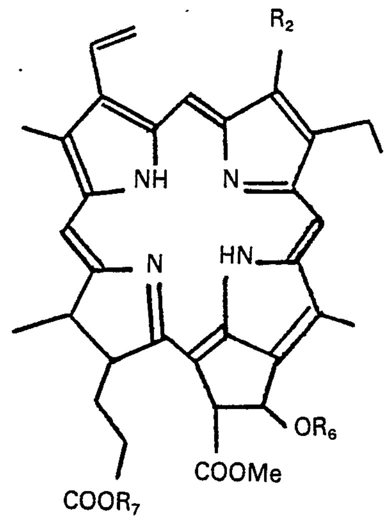

(Ia)

in der

$R_2$ $CH_3$ oder $CH_2OH$ bedeutet,

$R_6$ H oder Ac bedeutet, und

$R_7$ H oder ein Alkalimetallsalz, wie Na und K, bedeutet.

3. Pheophorbid-Derivate der allgemeinen Formel I, Ia oder II nach den Ansprüchen 1 oder 2, einschließlich 2-Desethenyl-2-(1-hydroxyethyl)-pheophorbid zur Verwendung als Arzneistoff.

4. Pheophorbid-Derivate der allgemeinen Formel I, Ia oder II nach den Ansprüchen 1 oder 2, einschließlich 2-Desethenyl-2-(1-hydroxyethyl)-pheophorbid zur Verwendung bei der Behandlung von Krebs.

5. Arzneimittel, enthaltend ein Pheophorbid-Derivat der allgemeinen Formel I, Ia oder II nach den Ansprüchen 1 oder 2, einschließlich 2-Desethenyl-2-(hydroxyethyl)-pheophorbid und einen pharmazeutisch verträglichen Träger.

**Revendications**

1. Dérivés de phéophorbide et sels de métaux alcalins de ces dérivés, représentés par la formule générale (I), et leurs dimères et les sels de métaux alcalins de ces dimères, représentés par la formule générale (II):

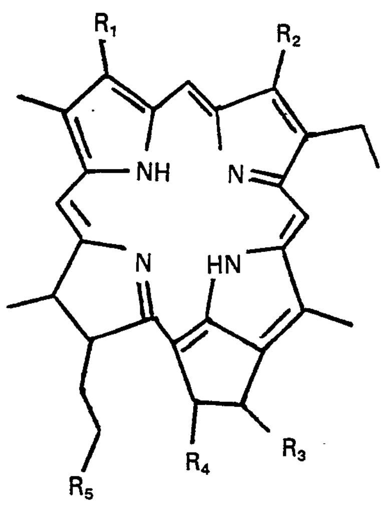

(I)

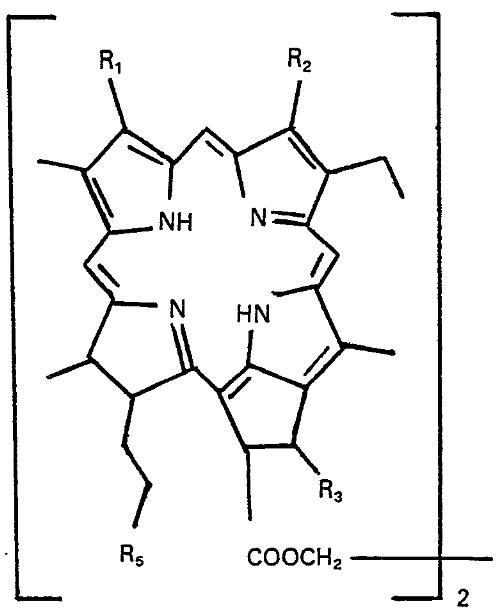

(II)

où:

$R_1$ représente $CH=CH_2$,

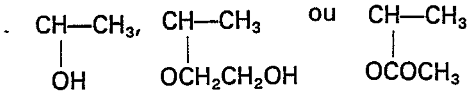

ou

R2 représente $CH_3$, CHO ou $CH_2OH$,

R3 représente =O, OH ou $OCOCH_3$;

R4 représente H, $COOCH_3$, $COOCH_2—CH_2OH$, $COOCH_2CH_2OCOCH_3$ ou un résidu CONH— d'un acide aminé; et

$R_5$ représente COOH, COO-alcali, et si $R_4$ représente un résidu CONH— d'un acide aminé, $R_5$ peut représenter en outre $COOCH_3$, à la condition cependant que desdits dérivés et sels alcalins soient exclus le phéophorbide, le pyrophéophorbide et le déséthényl-2 (hydroxy-1 éthyl)-2 phéophorbide.

2. Dérivés de phéophorbide et leurs sels de métaux alcalins selon la revendication 1, représentés par la formule générale (1a):

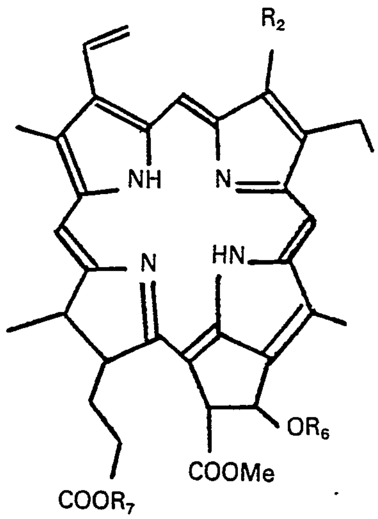

(Ia)

où:

$R_2$ représente $CH_3$ ou $CH_2OH$;

$R_6$ représente H ou Ac; et

$R_7$ représente H ou un métal alcalin tel que Na et K.

3. Dérivés de phéophorbide représentés par la formule générale (I), (Ia), ou (II) selon l'une des revendications 1 et 2, incluant le déséthényl-2 (hydroxy-1 éthyl)-2 phéophorbide, en vue d'une utilisation comme médicament.

4. Dérivés de phéophorbide représentés par la formule générale (I), (Ia) ou (II) selon l'une des revendications 1 et 2, incluant le déséthényl-2 (hydroxy-1 éthyl)-2 phéophorbide, en vue d'une utilisation dans le traitement du cancer.

5. Compositions pharmaceutiques contenant un dérivé de phéophorbide représenté par la formule générale (I), (Ia) ou (II) selon l'une des revendications 1 et 2, incluant le déséthényl-2 (hydroxy-1 éthyl)-2 phéophorbide et un support pharmaceutiquement acceptable.

Fig. 1

I ——— physiological saline
II – – – – cancer
III –·–·– liver
IV –··–··– kidney
V - - - - - plasma

after 24 hours

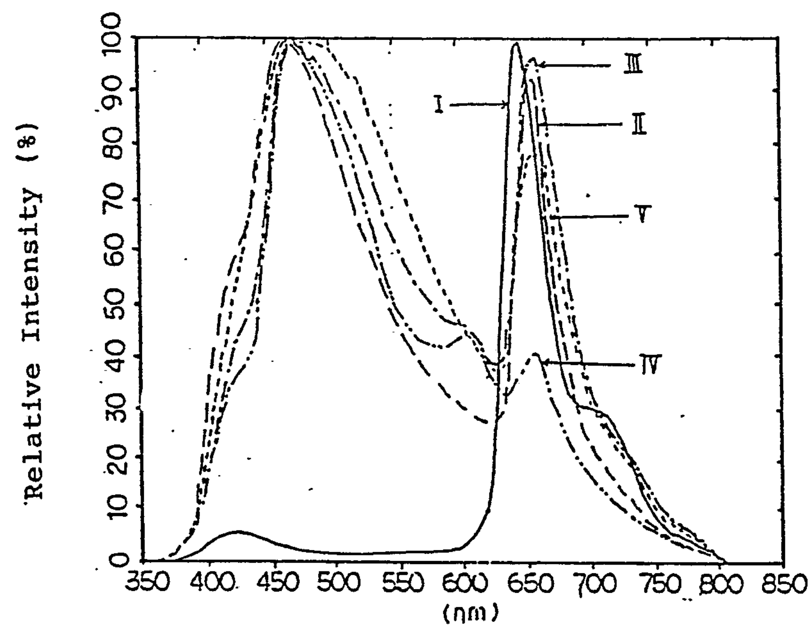


after 48 hours

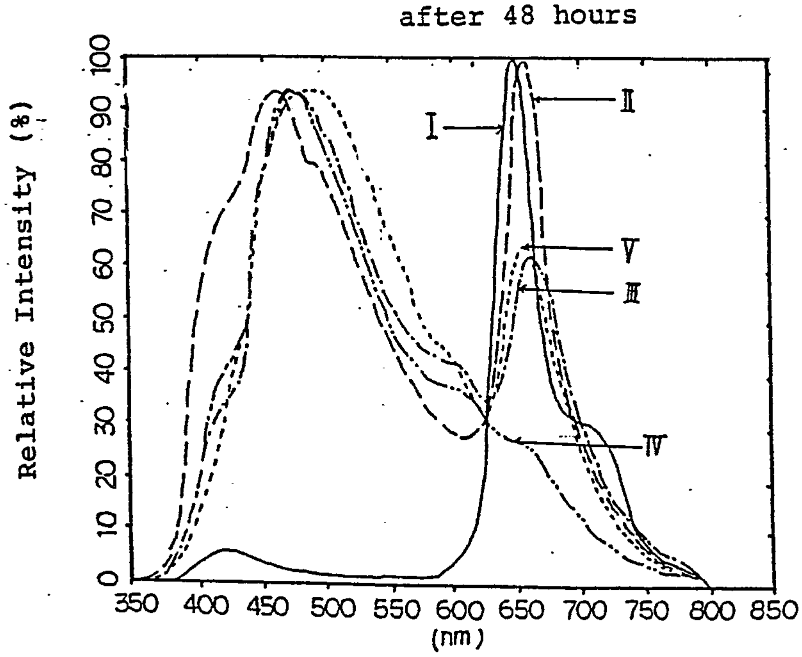

Fig. 2

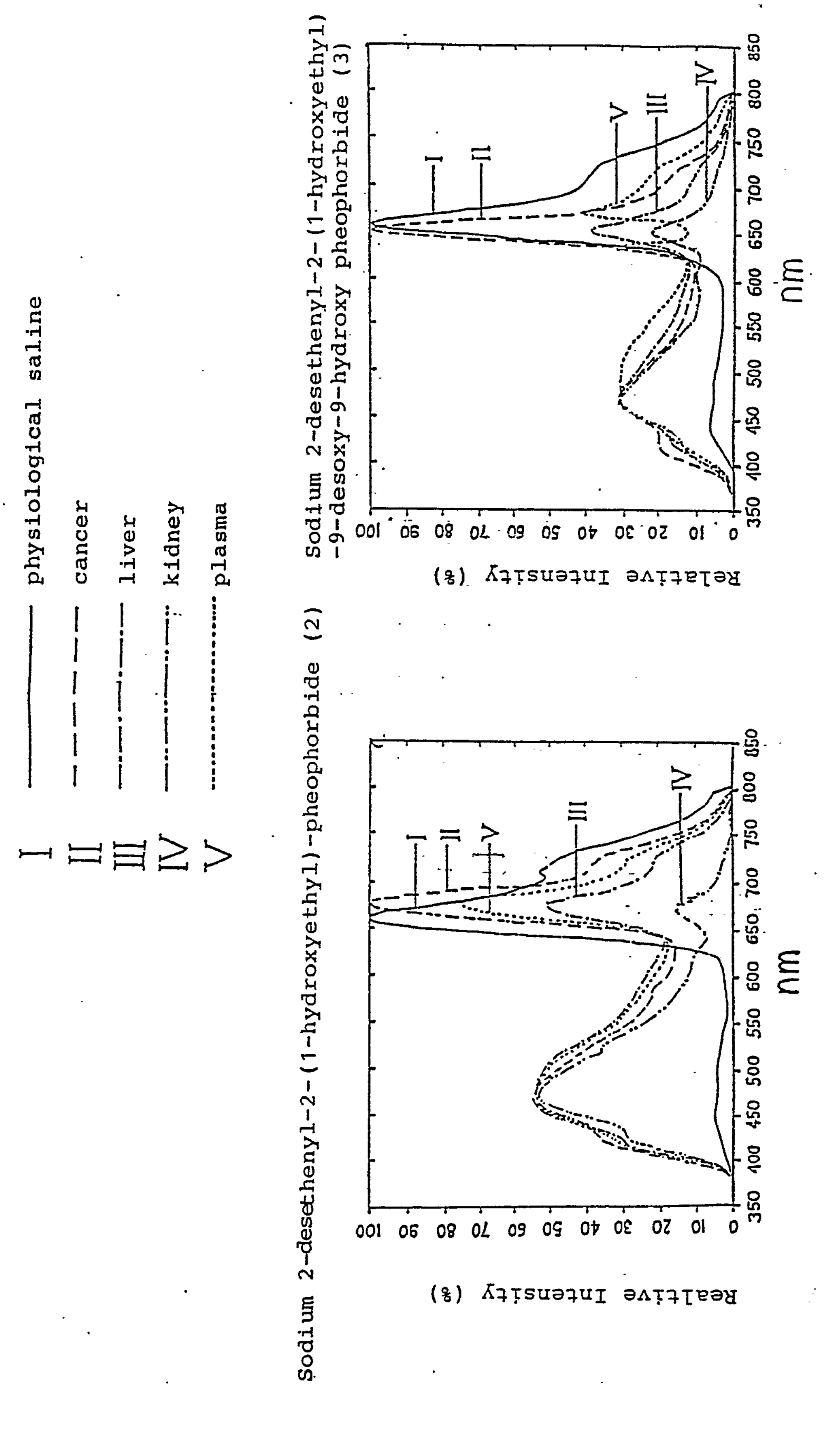

I — physiological saline
II — cancer
III — liver
IV — kidney
V — plasma
Sodium 2-desethenyl-2-(1-hydroxyethyl)-pheophorbide (2)
Realtive Intensity (%)
nm
Sodium 2-desethenyl-2-(1-hydroxyethyl)-9-desoxy-9-hydroxy pheophorbide (3)
Relative Intensity (%)
nm

Sodium 2-desethenyl-2-(1-acetyloxyethyl)-9-desoxo-9-acetyloxy-pheophorbide (4)

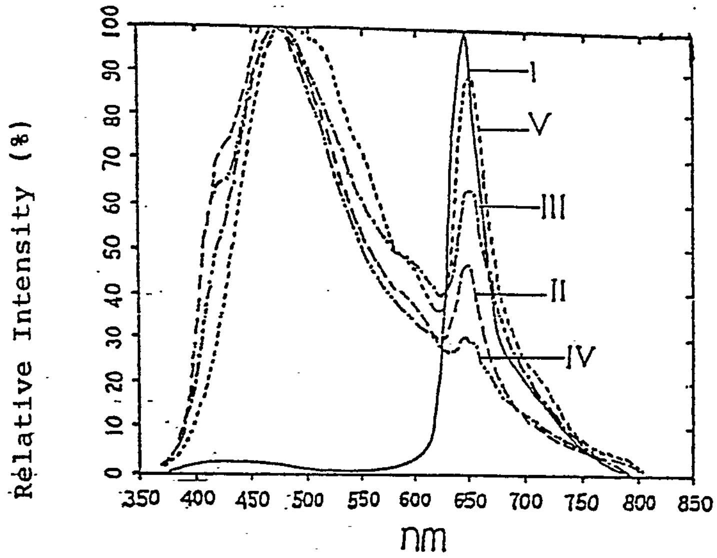


Sodium ethyleneglycol mono-10b-pheophorbate (8)

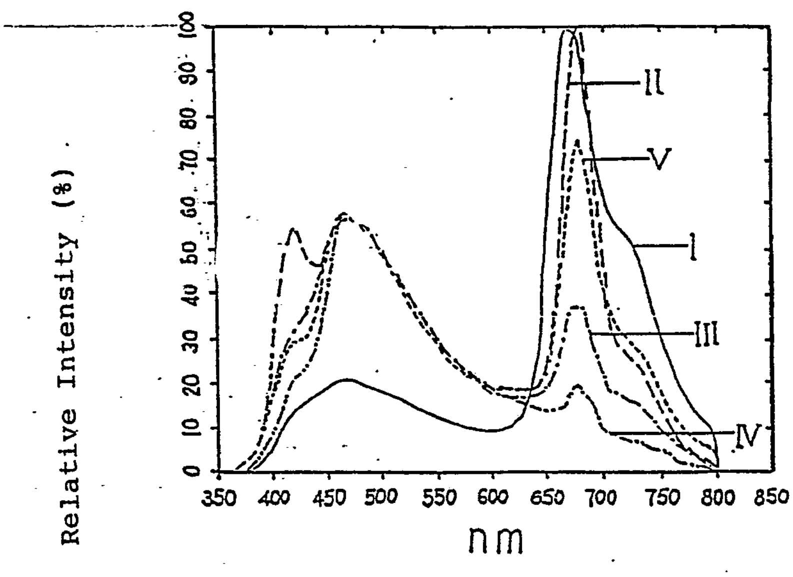

Sodium-N-phenylalanine-10b-methyl pheophorbidamide (9)

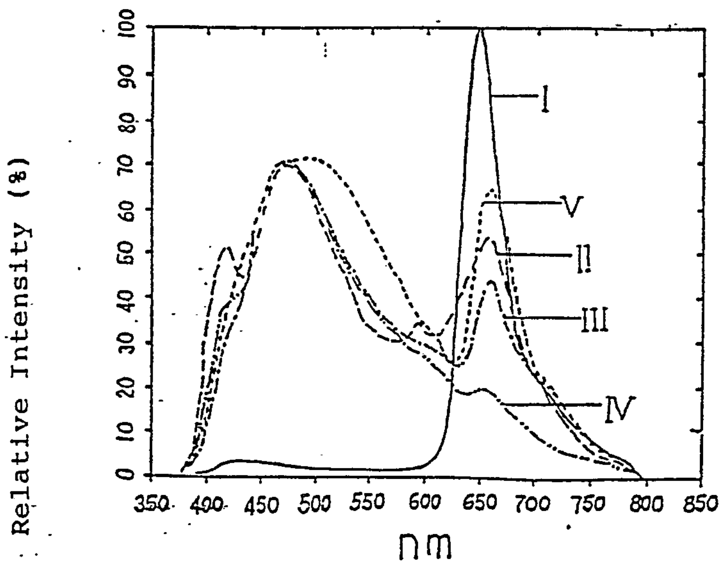


Sodium ethylene glycol di-10b-pheophorbate (10)

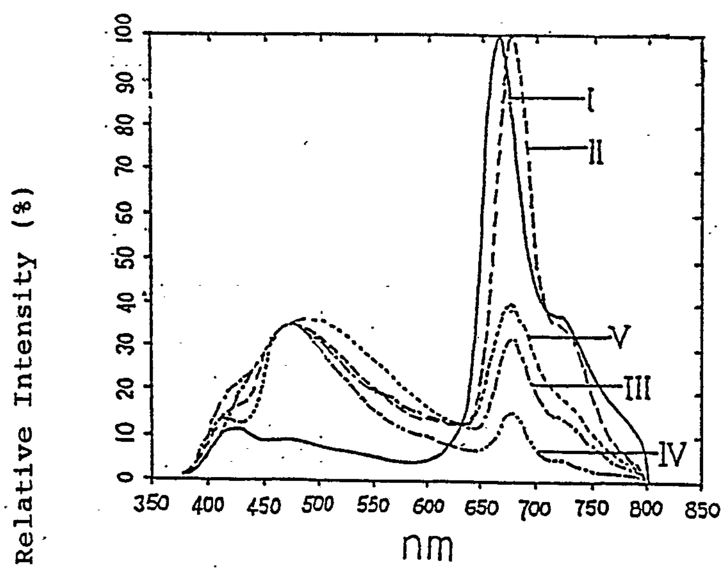

Fig. 3

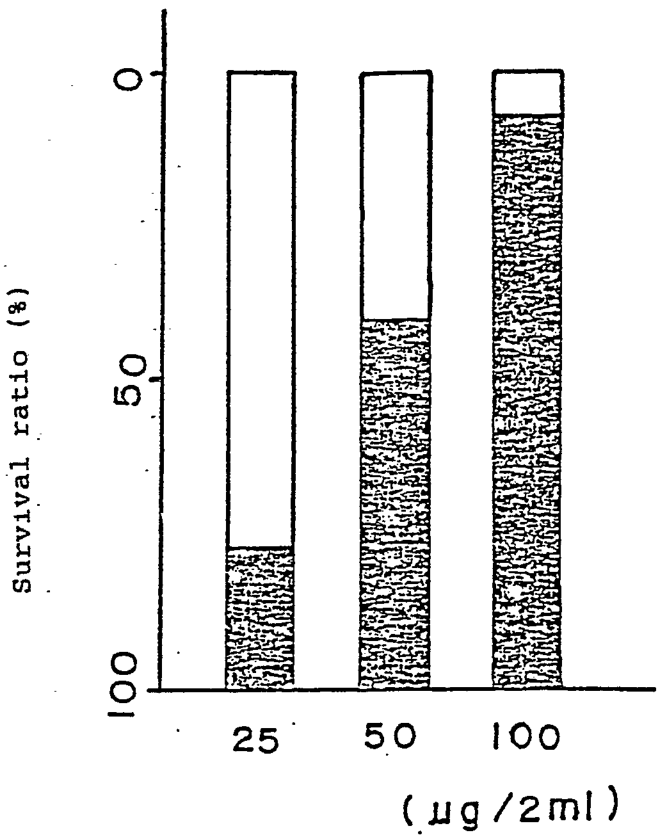

Fig. 4